(19) Europäisches Patentamt
European Patent Office
Office européen des brevets

(11) Veröffentlichungsnummer: **0 291 852**
**A1**

(12) # EUROPÄISCHE PATENTANMELDUNG

(21) Anmeldenummer: 88107592.3

(51) Int. Cl.[4]: **C07D 249/08 , A01N 43/653**

(22) Anmeldetag: 11.05.88

(30) Priorität: 20.05.87 DE 3716847
04.03.88 DE 3807041

(43) Veröffentlichungstag der Anmeldung:
23.11.88 Patentblatt 88/47

(84) Benannte Vertragsstaaten:
AT BE CH DE ES FR GB GR IT LI NL SE

(71) Anmelder: BASF Aktiengesellschaft
Carl-Bosch-Strasse 38
D-6700 Ludwigshafen(DE)

(72) Erfinder: Lauer, Manfred, Dr.
Horst-Schork-Strasse 83
D-6700 Ludwigshafen(DE)
Erfinder: Sauter, Hubert, Dr.
Neckarpromenade 20
D-6800 Mannheim 1(DE)
Erfinder: ROeser, Karl, Dr.
Muldweg 11
D-6945 Hirschberg(DE)
Erfinder: Jung, Johann, Prof. Dr.
Hardenburgstrasse 19
D-6703 Limburgerhof(DE)
Erfinder: Rademacher, Wilhelm, Dr.
Austrasse 1
D-6703 Limburgerhof(DE)
Erfinder: Will, Wolfgang, Dr.
Im Buegen 12
D-6719 Kirchheim(DE)

(54) **Triazolverbindungen, Verfahren zu ihrer Herstellung und Verfahren zur Regulierung des Pflanzenwachstums.**

(57) Triazolverbindungen der Formeln Ia und Ib

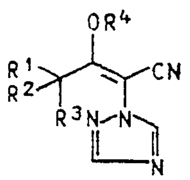

Ia

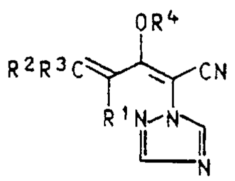

Ib

in denen

$R^1$ Wasserstoff, Cyclopropyl, unsubstituiertes $C_1$-$C_4$-Alkyl oder $C_2$-$C_4$-Alkinyl oder durch Methyl, Ethyl, Ethenyl, Ethinyl, Hydroxyl, Fluor, Chlor, Brom oder $C_1$-$C_4$-Alkoxy substituiertes $C_1$-$C_4$-Alkyl oder $C_2$-$C_4$-Alkinyl bedeutet oder auch für eine $C_1$-$C_4$-Alkoxy-, $C_2$-$C_4$-Alkenyloxy- und $C_2$-$C_4$-Alkinyloxygruppe steht

$R^2$, $R^3$ unabhängig voneinander Wasserstoff, lineares oder verzweigtes $C_1$-$C_3$-Alkyl bedeuten, wobei die Reste $R^2$ und $R^3$ auch zusammen einen gegebenenfalls Brückenglieder aufweisenden carbocyclischen Ring

EP 0 291 852 A1

mit bis zu sechs Gliedern in jedem Ringsystem bilden können und mit der Maßgabe, daß in der Formel Ia mindestens zwei der Reste $R^1$-$R^3$ von Wasserstoff verschieden sind und
$R^4$ Wasserstoff, einen Acylrest CO-$R^5$ oder einen Sulfonylrest $SO_2R^5$, wobei $R^5$ für lineares oder verzweigtes $C_1$-$C_8$Alkyl oder gegebenenfalls substituiertes Phenyl steht, bedeutet oder ein Metallkation oder Ammonium darstellt,

ein Verfahren zur Herstellung der Azole der Formel I und ihre Verwendung als Mittel des Pflanzenwachstums.

# Triazolverbindungen, Verfahren zu ihrer Herstellung und Verfahren zur Regulierung des Pflanzenwachstums

Die vorliegende Erfindung betrifft Triazolverbindungen, Mittel, welche diese Triazolverbindungen enthalten, Verfahren zu ihrer Herstellung und Verfahren zur Regulierung des Pflanzenwachstums mit diesen Triazolverbindungen.

Es ist bekannt, daß bestimmte, den erfindungsgemäßen Verbindungen ähnliche Triazolverbindungen als Zwischenprodukte zur Herstellung von Herbiziden geeignet sein können (DE OS 34 23 101).

Weiterhin ist bekannt, daß 2-Chlorethyltrimethylammoniumchlorid, (J. Biol. Chem. 235, 475 (1960)) pflanzenwachstumsregulierende Eigenschaften bei Getreide und anderen Kulturpflanzen aufweist. Ebenso ist es bekannt, daß triazolsubstituierte Alkohole (DE OS 27 37 489, 24 07 143) das Pflanzenwachstum beeinflussen.

Bei der Anwendung der bekannten Mittel zur Regulierung des Pflanzenwachstums ist die Wirkung vor allem bei niedrigen Aufwandmengen und Konzentrationen oft nicht ausreichend.

Es bestand daher die Aufgabe, neue Verbindungen zur Verfügung zu stellen, die das Pflanzenwachstum, insbesondere bei niedrigen Aufwendungen, wirksam regulieren können.

Es wurde nun überraschend gefunden, daß Triazole der Formel Ia, Ib

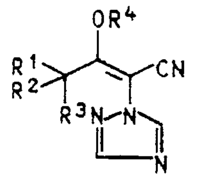

Ia

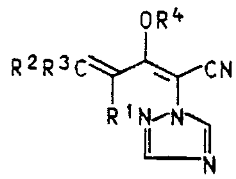

Ib

in denen

$R^1$ Wasserstoff, Cyclopropyl, unsubstituiertes $C_1$-$C_4$-Alkyl oder $C_2$-$C_4$-Alkinyl oder durch Methyl, Ethyl, Ethenyl, Ethinyl, Hydroxyl, Fluor, Chlor, Brom oder $C_1$-$C_4$-Alkoxy substituiertes $C_1$-$C_4$-Alkyl oder $C_2$-$C_4$-Alkinyl bedeutet oder auch für eine $C_1$-$C_4$-Alkoxy-, $C_2$-$C_4$-Alkenyloxy- und $C_2$-$C_4$-Alkinyloxygruppe steht

$R^2$, $R^3$ unabhängig voneinander Wasserstoff, lineares oder verzweigtes $C_1$-$C_3$-Alkyl bedeuten, wobei die Reste $R^2$ und $R^3$ gegebenenfalls zusammen einen gegebenenfalls Brückenglieder aufweisenden carbocyclischen Ring mit bis zu sechs Gliedern in jedem Ringsystem bilden können und mit der Maßgabe, daß in der Formel Ia mindestens zwei der Reste $R^1$-$R^3$ von Wasserstoff verschieden sind und

$R^5$ Wasserstoff, einen Acylrest CO-$R^5$ oder einen Sulfonylrest $SO_2$-$R^5$, wobei $R^4$ jeweils für lineares oder verzweigtes $C_1$-$C_8$ Alkyl oder gegebenenfalls substituiertes Phenyl steht, bedeutet oder ein Metallkation oder Ammonium darstellt,

den z.B. in DE-OS 27 37 489 und DE-OS 24 07 143 beschriebenen Mitteln und anderen Verbindungen, die bislang verwendet worden sind, in ihrer Wirkung überlegen sind.

$C_1$-$C_4$-Alkyl oder $C_2$-$C_4$-Alkinylgruppen in der Definition von $R^1$ sind beispielsweise Methyl, Ethyl, Propyl, Butyl, Ethinyl, Prop-2-inyl, Prop-1-inyl, But-1-inyl, But-2-inyl oder But-3-inyl. $C_1$-$C_4$-Alkoxy bedeutet beispielsweise Methoxy, Ethoxy, Propoxy, Isopropoxy, Butoxy, iso-Butoxy, sec. Butoxy oder tert.-Butoxy. $C_2$-$C_4$-Alkenyl bzw. Alkinyloxy sind z.B. Ethenyl-, Ethinyl-, Prop-2-enyl-, Prop-2-inyl-, Prop-1-enyl-, But-1-enyl-, But-1-inyl-, But-2-enyl-, But-3-enyl- oder But-3-inyloxy.

In der Definition von $R^2$, $R^3$ steht $C_1$-$C_3$-Alkyl z.B. für Methyl, Ethyl, Propyl, iso-Propyl. Die Reste $R^2$ und $R^3$ können auch miteinander zu einem carbocylischen System verknüpft sein, z.B. zu einem Ring mit 3 bis 6 C-Atomen wie Cyclopropyl, Cyclobutyl, Cyclopentyl oder Cyclohexyl. Dieser Ring kann zusätzlich z.B. durch eine $C_1$- oder $C_2$-Brücke überbrückt sein, so daß ein bicyclischer Rest wie Bicyclo[2.2.1]heptanyl (Norbornyl), Bicyclo[2.2.2]octanyl oder Bicyclo[2.1.1]hexanyl gebildet wird.

Beispielhaft seien für die Gruppe $CR^1R^2R^3$ 2-Propyl, tert-Butyl, 1-Methylpropyl, 1,1-Dimethylpropyl, 1,1-Dimethylbutyl, 1-Methyl-1-ethylpropyl, 1-Ethylpropyl, 1-Ethyl-propen-1-yl, Cyclopentyl, Cyclohexyl, 1-Methyl-1-methoxyethyl oder 1-Methyl-1-Ethoxyethyl genannt. Bevorzugt sind Verbindungen, in denen $R^1$ und $R^2$ eine Ethylgruppe bedeuten und $R^3$ für Wasserstoff oder Methyl steht.

$R^4$ steht für Kationen der Alkali- oder Erdalkalimetalle, z. B. Natrium, Kalium oder Magnesium,

Ammonium unter Einschluß der Kationen von organischen Aminen wie z. B. Methylamin, Dimethylamin, Trimethylamin, Diisopropylamin, N-Methyldiisopropylamin, N-Ethyldiisopropylamin, Dicyclohexylamin, Piperidin, N-Methylpiperidin, das N,N-Dimethyl piperidiniumion, N-Methyl-N-hexylamin und Morpholin oder aber für einen Acylrest CO-$R^5$ oder einen Sulfonylrest $SO_2R^5$.

Im Acylrest CO-$R^5$ oder Sulfonylrest $SO_2R^5$ steht $R^5$ für $C_1$- bis $C_8$-Alkyl wie Methyl, Ethyl, Propyl, Isopropyl, Butyl, iso-Butyl, sec.-Butyl, tert.-Butyl, Pentyl, iso-Pentyl, Hexyl, iso-Hexyl, Heptyl, iso-Heptyl, Octyl oder iso-Octyl oder für Phenyl oder substituiertes Phenyl. Als Substituenten am Phenylring kommen z.B. Halogen wie Fluor, Chlor, Brom, Iod, $C_1$-$C_4$-Alkyl, $C_2$-$C_4$-Alkenyl, $C_1$-$C_4$-Alkoxy oder Cyano in Betracht. Der Phenylring kann 1 bis 5, insbesondere 1 bis 3 inerte Substituenten tragen.

Triazolverbindungen der Formel I, in denen $R^1$, $R^2$ und $R^3$ die oben angegebene Bedeutung haben und in denen $R^4$ Wasserstoff bedeutet, werden durch Acylierung von Triazolylacetonitril mit Acylierungsmitteln der Formel $R^1R^2R^3CCOX$ hergestellt, wobei $R^1$-$R^3$ die oben angegebene Bedeutung besitzen und X für Chlor, Brom oder Iod steht. Aus wirtschaftlichen Gründen wird das Chlorid bevorzugt.

Geeignete Acylierungsmittel sind beispielsweise Propionsäurechlorid, Buttersäurechlorid, Isobuttersäurechlorid, 2-Methylbuttersäurechlorid, Pivalinsäurechlorid, 2,2-Dimethylbuttersäurechlorid, 2-Ethylbuttersäurechlorid, 2,2-Methyl-2-ethylbuttersäurechlorid, 2-Methylvaleriansäurechlorid, 2,2-Dimethylvaleriansäurechlorid, 2-Ethylvaleriansäurechlorid, 2-Methyl-2-ethyl-valeriansäurechlorid, 2,2-Diethylvaleriansäurechlorid, 2-Isopropylpropionsäurechlorid, 2-Methoxy-propionsäurechlorid, 2-Methoxy-2-methylpropionsäurechlorid, Cyclopentylcarbonsäurechlorid und 1-Methyl-cyclopentylcarbonsäurechlorid.

Die Umsetzung wird in Abwesenheit oder in Gegenwart eines geeigneten Verdünnungsmittels bei Temperaturen zwischen -50 und +150 °C durchgeführt. Geeignete Lösungsmittel sind z. B. Ether wie Diethylether, Methyl-tert.-butylether, Tetrahydrofuran oder Dioxan, Chlorierte Kohlenwasserstoffe wie Methylenchlorid, aromatische Kohlenwasserstoffe wie Toluol oder Xylol, Ketone wie Aceton und Alkohole wie tert.-Butanol sowie Dimethylformamid oder Dimethylsulfoxid.

Als Basen oder Säurefänger können beispielsweise Hydroxide von Alkali- und Erdalkalimetallen wie Natriumhydroxid, Kaliumhydroxid und Calciumhydroxid, Alkoholate von Alkali- und Erdalkalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali- oder Erdalkalicarbonate wie Natriumcarbonat, Kaliumcarbonat oder Calciumcarbonat; aliphatische Amine wie Dimethylamin, Triethylamin oder Diisopropylamin; heterocyclische Amine wie Piperidin, Piperazin oder Pyrrolidin; aromatische Amine wie Pyridin oder Pyrrol in stöchiometrischen Mengen oder im Überschuß verwendet werden.

Die Verbindungen Ia und Ib fallen als Z/E Isomerengemische an, die im Falle der Hydroxyverbindungen über die entsprechende Ketoform im Sinne einer Keto-Enol-Tautomerie im Gleichgewicht stehen können. Die jeweiligen Anteile von Z- und E-Enol und der tautomeren Ketoverbindungen hängen stark von Umgebungseinflüssen wie Temperatur oder Lösungsmittel ab. Die einzelnen Isomere wie ihre Gemische sind Gegenstand der vorliegenden Erfindung.

Triazolverbindungen der Formel Ia oder Ib, in denen $R^4$ nicht Wasserstoff, sondern einen Acylrest CO-$R^5$ bedeutet, wobei $R^5$ die oben angegebene Bedeutung hat, können in einem einstufigen Reaktionsschritt ähnlich wie bereits für die freie Hydroxyverbindung beschrieben durch Acylierung von Triazolylacetonitril erhalten werden, allerdings in Gegenwart von mindestens zwei Equivalenten an Acylchlorid. Vorteilhaft ist für $R^4$ = CO-$R^5$ bisweilen eine zweistufige Reaktionsführung, in denen die Verbindung durch Acylierung der Verbindung I mit $R^4$ = H durchgeführt wird. Als Acylierungsmittel können die voranstehend beschriebenen Verbindungen, z.B. Acetylchlorid, Pivaloylchlorid, 2-Ethylbuttersäurechlorid, 2-Methylbuttersäurechlorid oder Benzoylchlorid verwendet werden.

Triazolverbindungen der Formel Ia oder Ib, in denen $R^4$ nicht Wasserstoff, sondern einen Sulfonylrest $SO_2R^5$ bedeutet, wobei $R^5$ die obengenannte Bedeutung hat, werden durch Umsetzung der entsprechenden Hydroxyverbindungen I ($R^4$ = H) mit Sulfonsäurehalogeniden X-$SO_2$-$R^5$, worin X Halogen wie Chlor, Brom oder Iod bedeutet und $R^5$ die obengenannte Bedeutung hat oder entsprechend substituierten Sulfonsäureanhydriden in Gegenwart einer Base hergestellt.

Als Basen oder Säurefänger können die an sich üblichen oben angegebenen Verbindungen wie Alkali- oder Erdalkaliverbindungen z.B. Carbonate, Hydroxide oder Alkoholate, tertiäre Amine, Pyridin oder substituiertes Pyridin verwendet werden. Beispielsweise seien Natriumcarbonat, Natriumhydroxid, Kaliumhydroxid, Kalium-tert.-butylat, Trimethylamin, Triethylamin, N-Methylpiperidin oder Pyridin genannt. Vorteilhaft kann die Umsetzung in Gegenwart katalytischer Mengen 4-N,N'-Dimethylaminopyridin vorgenommen werden.

Die Base kann in stöchiometrischen Mengen oder im Überschuß, vorteilhaft in Mengen von 1 bis 2 mol pro Mol der Hydroxyverbindung I mit $R^4$ = H eingesetzt werden.

Das Sulfonsäurehalogenid bzw. -anhydrid wird üblicherweise in äquimolaren Mengen, bezogen auf die Hydroxyverbindung, verwendet. Ein Über- oder Unterschuß, z.B. 0,5 bis 2 mol, ist ebenfalls möglich.

Die Umsetzung erfolgt in Gegenwart oder Abwesenheit eines Lösungsmittels bei Temperaturen von -50 bis +150 °C. Geeignete Lösungs- oder Verdünnungsmittels sind z.B. Ether wie Tetrahydrofuran, Diethylether, Methyl-tert.-butylether, Dioxan; chlorierte Kohlenwasserstoffe wie Methylenchlorid, aromatische Kohlenwasserstoffe wie Toluol oder Xylol, Ketone, z.B. Aceton, Alkohole wie tert.-Butanol, Amide wie Dimethylformamid oder Sulfoxide wie Dimethylsulfoxid.

Triazolverbindungen der Formel Ia oder Ib, in denen $R^4$ nicht Wasserstoff, sondern ein Metall- oder Ammoniumkation bedeutet, werden durch Umsetzung der entsprechenden Hydroxyverbindung I und einer geeigneten Base gegebenenfalls in Gegenwart eines geeigneten Verdünnungsmittels hergestellt. Als Basen eignen sich z. B. Natron- oder Kalilauge, Dimethylamin, Diethylamin, Trimethylamin, Triethylamin, Ethanolamin, Diethanolamin, Triethanolamin, Diisopropylamin, Diisopropylethylamin, Piperidin, Dicyclohexylamin, N-Methylpiperidin, Morpholin oder 2,6-Dimethylmorpholin. Die Basenmenge ist nicht besonders kritisch, es können equimolare Mengen oder ein Überschuß der Base, bezogen auf die Hydroxyverbindungen, z.B. 1 bis 2 mol pro Mol I, verwendet werden.

Herstellungsbeispiel 1

Zu einer Lösung von 68 g (0,5 M) 2-Ethylbuttersäurechlorid und 54 g (0,5 mol) Triazolylacetonitril in 500 ml Tetrahydrofuran werden bei -20 °C portionsweise 130 g (1,2 mol) Kalium-tert.-butylat zugegeben. Man läßt auf Raumtemperatur kommen, rührt 12 Stunden nach, engt ein und verdünnt mit 1000 ml Wasser. Nach Extraktion von Verunreinigungen mit Methyl-tert.-butylether wird die wäßrige Phase mit verdünnter Salzsäure angesäuert und mehrmals mit Methylenchlorid extrahiert. Die organische Phase wird eingeengt und das entstandene 1-Cyano-1-(1,2,4-triazol-1-yl)-3-ethyl-1-penten-2-ol aus Ethanol/Wasser umkristallisiert. Ausbeute 73 g (71 % d. Th.) Schmp. 98 - 103 °C.

In der nachstehenden Tabelle ist die erhaltene Verbindung als Nr. 49 aufgeführt.

Herstellungsbeispiel 2

6 g (29 mmol) der wie vorstehend erhaltenen Verbindung 49 werden in Pyridin gelöst und in Gegenwart einer katalytischen Menge an Dimethylaminopyridin mit 35 ml (30 mmol) Benzoylchlorid 5 Stunden bei 50 °C umgesetzt. Nach Einengen wird das Umsetzungsprodukt an einer Säule chromatographisch gereinigt. Ausbeute: 0,5 g (14 %) an 2-Benzoyl-1-cyano-1-(1,2,4-triazol-1-yl)-3-ethyl-1-penten als Z/E Isomerengemisch (Nr. 89 in Tabelle 1)

IR: 2970, 1757, 1505, 1205, 1086 $cm^{-1}$

Herstellungsbeispiel 3

Zu einer Suspension von 50 g (0,24 mol)
1-Cyano-1-(1,2,4-triazol-1-yl)-3-ethyl-1-penten-2-ol in 300 ml Toluol werden nach Zugabe von 0,5 g 4-N,N'-Dimethylaminopyridin 50 g (0,25 mol) p-Toluolsulfonsäurechlorid zugegeben. Anschließend werden 50 ml (0,36 mol) Triethylamin zugetropft. Man läßt 40 Stunden bei Raumtemperatur rühren, schüttelt mit Wasser aus, engt ein und läßt das Produkt auskristallisieren. Nach Umkristallisieren aus Ethanol werden 60 g (68 %) 2-(4-Methylphenylsulfonyl)-1-cyano-1-(1,2,4-triazol-yl)-3-ethyl-1-penten als Z,E-Isomerengemisch erhalten. Schmp. 78-87 °C

$C_{17}H_{23}N_4O_3S$
ber. C 56,7 H 5,6 N 15,5 S 8,9
gef. C 56,7 H 5,8 N 15,6 S 8,8

In der nachstehenden Tabelle ist die erhaltene Verbindung als Nr. 109 aufgeführt.

Die nachfolgend tabellarisch wiedergegebenen beispielhaften Wirkstoffe wurden, soweit physikalische Angaben beigefügt sind, jeweils hergestellt. Die übrigen können mit den angegebenen Maßnahmen aus entsprechenden Vorprodukten erhalten werden; sie lassen aufgrund von Äquivalenzüberlegungen eine vergleichbare Wirkung erwarten.

Tabelle 1

Triazolverbindungen der Formel

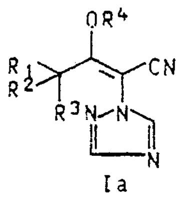

Ia

| Nr. | $R^1$ | $R^2$ | $R^3$ | $R^4$ | Schmp./IR ($cm^{-1}$) |
|---|---|---|---|---|---|
| 1 | $CH_3$- | $CH_3$ | H | H | 78 - 80°C |
| 2 | $C_2H_5$- | $CH_3$ | H | H | 87°C |
| 3 | $CH_2$=CH- | $CH_3$ | H | H | |
| 4 | CH≡C | $CH_3$ | H | H | |
| 5 | $CH_3CH_2CH_2$- | $CH_3$ | H | H | 2961, 2935, 1633, 1508 |
| 6 | $(CH_3)_2CH$- | $CH_3$ | H | H | 117°C |
| 7 | Cyclopropyl | $CH_3$ | H | H | |
| 8 | $CH_2$=CH-$CH_2$- | $CH_3$ | H | H | |
| 9 | CH≡C-$CH_2$- | $CH_3$ | H | H | |
| 10 | $CH_3CH_2CH_2CH_2$- | $CH_3$ | H | H | |
| 11 | $(CH_3)_2CHCH_2$- | $CH_3$ | H | H | |
| 12 | $CH_3CH(CH_3)CH_2$- | $CH_3$ | H | H | |
| 13 | $(CH_3)_3C$- | $CH_3$ | H | H | |
| 14 | $CH_2$=$CHCH_2CH_2$- | $CH_3$ | H | H | |
| 15 | CH≡C-$CH_2CH_2$- | $CH_3$ | H | H | |
| 16 | $HOCH_2$- | $CH_3$ | H | H | |
| 17 | $FCH_2$- | $CH_3$ | H | H | |
| 18 | $ClCH_2$- | $CH_3$ | H | H | |
| 19 | $CH_3O$ | $CH_3$ | H | H | 117 - 118°C |
| 20 | $CH_3OCH_2$- | $CH_3$ | H | H | |
| 21 | $CH_3CH_2OCH_2$- | $CH_3$ | H | H | |
| 22 | $CH_2$=$CHCH_2OCH_2$- | $CH_3$ | H | H | |
| 23 | CH≡$CCH_2OCH_2$- | $CH_3$ | H | H | |
| 24 | $CH_3CH_2CH_2CH_2OCH_2$- | $CH_3$ | H | H | |
| 25 | $CH_3$- | $CH_3$ | $CH_3$ | H | 2976, 1738, 1506, 1277 |
| 26 | $C_2H_5$- | $CH_3$ | $CH_3$ | H | 2973, 1736, 1505, 1279 |
| 27 | $CH_2$=CH- | $CH_3$ | $CH_3$ | H | |
| 28 | CH≡C- | $CH_3$ | $CH_3$ | H | |
| 29 | $CH_3CH_2CH_2$- | $CH_3$ | $CH_3$ | H | 2965, 1505, 1474, 1279 |
| 30 | $(CH_3)_2CH$- | $CH_3$ | $CH_3$ | H | |
| 31 | Cyclopropyl | $CH_3$ | $CH_3$ | H | |
| 32 | $CH_2$=$CHCH_2$- | $CH_3$ | $CH_3$ | H | |
| 33 | CH≡$CCH_2$- | $CH_3$ | $CH_3$ | H | |
| 34 | $CH_3CH_2CH_2CH_2$- | $CH_3$ | $CH_3$ | H | |

Tabelle 1 (zu Formel Ia) (Fortsetzung)

| Nr. | $R^1$ | $R^2$ | $R^3$ | $R^4$ | Schmp./IR ($cm^{-1}$) |
|---|---|---|---|---|---|
| 35 | $(CH_3)_2CHCH_2$- | $CH_3$ | $CH_3$ | H | |
| 36 | $CH_3CH(CH_3)CH_2$- | $CH_3$ | $CH_3$ | H | |
| 37 | $(CH_3)_3C$- | $CH_3$ | $CH_3$ | H | |
| 38 | $CH_2=CHCH_2CH_2$- | $CH_3$ | $CH_3$ | H | |
| 39 | $CH\equiv CCH_2CH_2$- | $CH_3$ | $CH_3$ | H | |
| 40 | $HOCH_2$- | $CH_3$ | $CH_3$ | H | xHCl→ 171 - 179°C |
| 41 | $FCH_2$- | $CH_3$ | $CH_3$ | H | |
| 42 | $ClCH_2$- | $CH_3$ | $CH_3$ | H | |
| 43 | $CH_3O$- | $CH_3$ | $CH_3$ | H | |
| 44 | $CH_3OCH_2$- | $CH_3$ | $CH_3$ | H | 2936, 1739, 1506, 1277 |
| 45 | $CH_3CH_2OCH_2$- | $CH_3$ | $CH_3$ | H | 2978, 1739, 1505, 1277, 1113 |
| 46 | $CH_2=CH-CH_2OCH_2$- | $CH_3$ | $CH_3$ | H | |
| 47 | $CH\equiv C-CH_2OCH_2$- | $CH_3$ | $CH_3$ | H | |
| 48 | $CH_3CH_2CH_2CH_2OCH_2$- | $CH_3$ | $CH_3$ | H | 2960, 1739, 1474, 1277 |
| 49 | $C_2H_5$- | $C_2H_5$ | H | H | 98 - 103°C |
| 50 | $CH_2=CH$- | $C_2H_5$ | H | H | |
| 51 | $CH\equiv C$- | $C_2H_5$ | H | H | |
| 52 | $CH_3CH_2CH_2$- | $C_2H_5$ | H | H | |
| 53 | $(CH_3)_2CH$- | $C_2H_5$ | H | H | |
| 54 | $CH_2=CHCH_2$- | $C_2H_5$ | H | H | |
| 55 | $CH\equiv CCH_2$- | $C_2H_5$ | H | H | |
| 56 | $CH_3CH_2CH_2CH_2$- | $C_2H_5$ | H | H | 59 - 61°C |
| 57 | $(CH_3)_2CHCH_2$- | $C_2H_5$ | H | H | |
| 58 | $CH_3CH(CH_3)CH_2$- | $C_2H_5$ | H | H | |
| 59 | $C_2H_5$- | $C_2H_5$ | $CH_3$ | H | 3112, 2969, 2207, 1520 |
| 60 | $CH_2=CH$- | $C_2H_5$ | $CH_3$ | H | |
| 61 | $CH\equiv C$- | $C_2H_5$ | $CH_3$ | H | |
| 62 | $CH_3CH_2CH_2$- | $C_2H_5$ | $CH_3$ | H | |
| 63 | $CH_3CH_2CH_2CH_2$- | $C_2H_5$ | $CH_3$ | H | |
| 64 | $C_2H_5$- | $C_2H_5$ | $C_2H_5$ | H | |
| 65 | $CH_3CH_2CH_2$- | $CH_3$ | $C_2H_5$ | H | |
| 66 | $CH_3CH_2CH_2CH_2$- | $CH_3$ | $C_2H_5$ | H | |
| 67 | $CH_3CH_2CH_2$- | n-$C_3H_7$ | H | H | 88 - 91°C |
| 68 | $CH_3CH_2CH_2$- | $CH_3$ | H | H | |
| 69 | $CH_3CH_2CH_2$- | $C_2H_5$ | H | H | |
| 70 | $CH_3CH_2CH_2$- | $C_3H_7$ | H | H | |
| 71 | H- | Cyclo-propyl | H | H | 2209, 1592, 1508, 1277 |
| 72 | $CH_3$- | Cyclo-propyl | H | H | 152 -156°C |

Tabelle 1 (zu Formel Ia)

| Nr. | $R^1$ | $R^2$ | $R^3$ | $R^4$ | Schmp./IR ($cm^{-1}$) |
|---|---|---|---|---|---|
| 73 | H- | Cyclo- | H | H | 2950, 1624, 1508, 1277 |
| | | butyl | | | |
| 74 | $CH_3$- | Cyclo- | H | H | |
| | | butyl | | | |
| 75 | H | Cyclo- | H | H | 81 - 82°C |
| | | pentyl | H | H | |
| 76 | $CH_3$ | Cyclo- | H | H | |
| | | pentyl | H | H | |
| 77 | H | Cyclo- | H | H | 121 - 125°C |
| | | hexyl | H | H | |
| 78 | $CH_3$ | Cyclohexyl | H | H | 2935, 1504, 1277, 1134 |
| 79 | H | Adamantyl | H | H | 114 -117°C |
| 80 | $CH_3$ | Adamantyl | H | H | |
| 81 | $C_2H_5$ | $C_2H_5$ | H | COH | |
| 82 | $C_2H_5$ | $C_2H_5$ | H | $COCH_3$ | 2970, 1757, 1505, 1205 |
| 83 | $C_2H_5$ | $C_2H_5$ | H | $COC_2H_5$ | 2967, 1785, 1506, 1087 |
| 84 | $C_2H_5$ | $C_2H_5$ | H | $COCH_2CH_3CH_3$ | |
| 85 | $C_2H_5$ | $C_2H_5$ | H | $COCH_2CH_2CH_2CH_3$ | |
| 86 | $C_2H_5$ | $C_2H_5$ | H | $COC(CH_3)_3$ | |
| 87 | $C_2H_5$ | $C_2H_5$ | H | $COCH(CH_3)CH_2CH_3$ | |
| 88 | $C_2H_5$ | $C_2H_5$ | H | $COCH(C_2H_5)_2$ | |
| 89 | $C_2H_5$ | $C_2H_5$ | H | 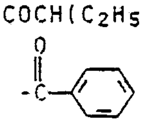 | 2970, 1757, 1505, 1205 |
| 90 | $C_2H_5$ | $C_2H_5$ | H | 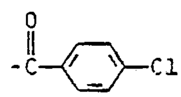 | 1758, 1593, 1204, 1089 |
| 91 | $C_2H_5$ | $C_2H_5$ | H | 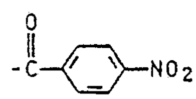 | |
| 92 | $C_2H_5$ | $C_2H_5$ | H | 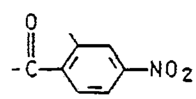 | |
| 93 | $C_2H_5$ | $C_2H_5$ | H | 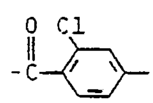 | 2957, 1770, 1585, 1201 |
| 94 | $CH_3$ | $C_2H_5$ | H | 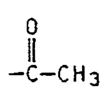 | 2970, 2937, 2217, 1633 |

Tabelle 1 (zu Formel Ia)

| Nr. | $R^1$ | $R^2$ | $R^3$ | $R^4$ | Schmp./IR (cm-1) |
|---|---|---|---|---|---|
| 95 | $CH_3$ | $CH_3$ | $CH_3$ | $SO_2CH_3$ | 1362, 1187, 1088, 795 |
| 96 | $CH_3$ | $CH_3$ | $CH_3$ | $SO_2$– 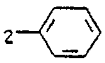 | |
| 97 | $CH_3$ | $CH_3$ | CH3 | $SO_2$– 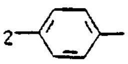 | |
| 98 | $CH_3$ | $CH_3$ | $CH_3$ | $SO_2$– 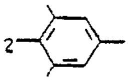 | |
| 99 | H | $CH_3$ | $C_2H_5$ | $SO_3CH_3$ | 60-65° C |
| 100 | H | $CH_3$ | $C_2H_5$ | $SO_2$– 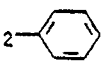 | |
| 101 | H | $CH_3$ | $C_2H_5$ | $SO_2$– 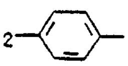 | |
| 102 | H | $CH_3$ | $C_2H_5$ | $SO_2$– 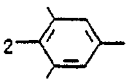 | 1371, 1176, 1210, 1053 |
| 103 | H | $CH_3$ | i-$C_3H_7$ | $SO_2CH_3$ | |
| 104 | H | $CH_3$ | i-$C_3H_7$ | $SO_2$– 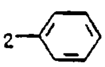 | |
| 105 | H | $CH_3$ | i-$C_3H_7$ | $SO_2$– 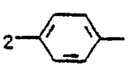 | 30-40° C |
| 106 | H | $CH_3$ | i-$C_3H_7$ | $SO_2$– 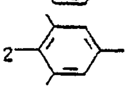 | |
| 107 | H | $C_2H_5$ | $C_2H_5$ | $SO_2CH_3$ | 1506, 1372, 1210, 1177 |
| 108 | H | $C_2H_5$ | $C_2H_5$ | $SO_2$– 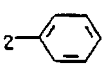 | |
| 109 | H | $C_2H_5$ | $C_2H_5$ | $SO_2$– 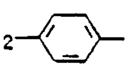 | 78-87° C |
| 110 | H | $C_2H_5$ | $C_2H_5$ | $SO_2$– 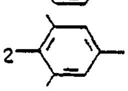 | 2968, 1054, 1370, 1175 |
| 111 | H | $C_2H_5$ | $C_2H_5$ | $SO_2$– 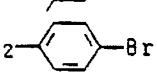 | 108-114° C |
| 112 | H | $C_2H_5$ | $C_2H_5$ | $SO_2$– 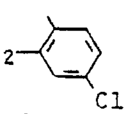 | 112-116° C |
| 113 | H | $C_2H_5$ | $C_2H_5$ | $SO_2$– 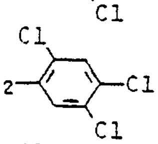 | 137-139° C |
| 114 | H | $C_2H_5$ | $C_2H_5$ | $SO_2$– 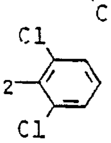 | 100-104° C |

Tabelle 2

Triazolverbindungen der Formel

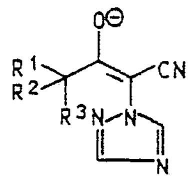

$R^4\oplus$

| Nr. | $R^1$ | $R^2$ | $R^3$ | $R^4$ | Schmp./IR ($cm^{-1}$) |
|---|---|---|---|---|---|
| 115 | $C_2H_5$ | $C_2H_5$ | H | $Na^{\oplus}$ | 95 - 100°C |
| 116 | $C_2H_5$ | $C_2H_5$ | H | $K^{\oplus}$ | |
| 117 | $C_2H_5$ | $C_2H_5$ | H | $NH_4^{\oplus}$ | |
| 118 | $C_2H_5$ | $C_2H_5$ | H | $CH_3NH_3^{\oplus}$ | |
| 119 | $C_2H_5$ | $C_2H_5$ | H | $(CH_3)_2NH_2^{\oplus}$ | |
| 120 | $C_2H_5$ | $C_2H_5$ | H | $(CH_3)_3NH^{\oplus}$ | |
| 121 | $C_2H_5$ | $C_2H_5$ | H | $(CH_4)N^{\oplus}$ | |
| 122 | $C_2H_5$ | $C_2H_5$ | H | $(C_2H_5)_2NH_2^{\oplus}$ | |
| 123 | $C_2H_5$ | $C_2H_5$ | H | $(C_2H_5)_3NH^{\oplus}$ | |
| 124 | $C_2H_5$ | $C_2H_5$ | H | $((CH_3)_2CH)NH_3^{\oplus}$ | |
| 125 | $C_2H_5$ | $C_2H_5$ | H | $((CH_3)_2CH)_2NH_2^{\oplus}$ | 2962, 2170, 1539, 1275 |
| 126 | $C_2H_5$ | $C_2H_5$ | H | $((CH_3)_2CH)_2NHCH_3^{\oplus}$ | |
| 127 | $C_2H_5$ | $C_2H_5$ | H | $((CH_3)_2CH)_2NHC_2H_5^{\oplus}$ | |
| 128 | $C_2H_5$ | $C_2H_5$ | H | Cyclohexyl-$NH_3^{\oplus}$ | |
| 129 | $C_2H_5$ | $C_2H_5$ | H | (Cyclohexyl)$_2$-$NH_2^{\oplus}$ | |
| 130 | $C_2H_5$ | $C_2H_5$ | H | 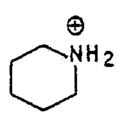 | |
| 131 | $C_2H_5$ | $C_2H_5$ | H | 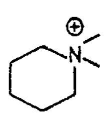 | |
| 132 | $C_2H_5$ | $C_2H_5$ | H | 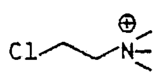 | $^1$H-NMR in $CDCl_3$ (ppm): 0,85(t); 3,2(d); 3,8(d); 4,1(t); 7,85(s); 8,55(s) |
| 133 | $C_2H_5$ | $C_2H_5$ | H | $(HOCH_2CH_2)_2NH_2^{\oplus}$ | 2961, 2175, 1537, 1457, 1403 |

Tabelle 2

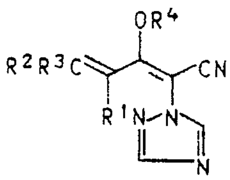

Ib

Triazolverbindungen der Formel Ib

| Nr. | $R^1$ | $R^2$ | $R^3$ | $R^4$ | Schmp./IR ($cm^{-1}$) |
|---|---|---|---|---|---|
| 134 | H | H | H | H | |
| 135 | H | $CH_3$ | H | H | |
| 136 | H | $CH_3$ | $CH_3$ | H | 118°C |
| 137 | H | $C_2H_5$ | H | H | |
| 138 | H | $C_2H_5$ | $CH_3$ | H | |
| 139 | H | $C_2H_5$ | $C_2H_5$ | H | |
| 140 | $CH_3$ | H | H | H | |
| 141 | $CH_3$ | $CH_3$ | H | H | 64 - 71°C |
| 142 | $CH_3$ | $CH_3$ | $CH_3$ | H | |
| 143 | $CH_3$ | $C_2H_5$ | H | H | |
| 144 | $CH_3$ | $C_2H_5$ | $C_2H_5$ | H | |
| 145 | $CH_3$ | $C_2H_5$ | $C_2H_5$ | H | |
| 146 | $C_2H_5$ | H | H | H | |
| 147 | $C_2H_5$ | $CH_3$ | H | H | 81 - 88°C |
| 148 | $C_2H_5$ | $CH_3$ | $CH_3$ | H | |
| 149 | $C_2H_5$ | $C_2H_5$ | H | H | |
| 150 | $C_2H_5$ | $C_2H_5$ | $CH_3$ | H | |
| 151 | $C_2H_5$ | $C_2H_5$ | $C_2H_5$ | H | |

Die Triazolderivate der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des 2Lagerns2 (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt.

Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Triazolderivate der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,01 bis 10 kg/ha, bevorzugt 0,05 bis 3 kg/ha, als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), N,N-Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, N,N-Dimethylformamid oder N-Methylpyrrolidon. Die Formulierungen enthalten im allgemeinen zwischen 0,1 und 95 Gew.% Wirkstoff, vorzugsweise zwischen 0,5 und 90 Gew.%.

Die Formulierungen bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I 20 Gewichtsteile der Verbindung des Beispiel 1 werden in 3 Gewichtsteilen des Natriumsalzes der Diisobutylanphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II 3 Gewichtsteile der Verbindung des Beispiels 133 werden mit 97 Gewichtsteilen feinteiligem Kaolin innig vermischt. Man erhält auf diese Weise ein Stäubemittel, das 3 Gew.% des Wirkstoffs enthält.

III 30 Gewichtsteile der Verbindung des Beispiels 110 werden in einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 20 Teile der Verbindung des Beispiels 109 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

V Man vermischt 90 Gewichtsteile der Verbindung des Beispiels 99 mit 10 Gewichtsteilen N-Methyl-$\alpha$-pyrrolidon und erhält eine Lösung, die zur Anwendung in Form kleinster Tropfen geeignet ist.

VI 20 Gewichtsteile der Verbindung des Beispiels 109 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VII 20 Gewichtsteile der Verbindung des Beispiels 25 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

VIII. 20 Gewichtsteile der Verbindung des Beispiels 25 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Die erfindungsgemäßen Mittel können in diesen Anwendungsformen auch zusammen mit anderen

Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit anderen Wachstumsregulatoren treten auch synergistische Effekte auf, d.h. die Wirksamkeit des Kombinationsproduktes ist größer als die addierten Wirksamkeiten der Einzelkomponenten.

Anwendungsbeispiele

Zur Bestimmung der wachstumsregulierenden Eigenschaft der Prüfsubstanzen wurden die Testpflanzen auf ausreichend mit Nährstoffen versorgtem Kultursubstrat in Kunststoffgefäßen (ca. 12,5 cm Durchmesser; Volumen ca. 500 ml) angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz A diente 2-Chlorethyltrimethylammoniumchlorid (CCC).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen. Mit den Tosylaten (Verbindungen 95 bis 114) wurden separate Versuchsreihen durchgeführt, so daß unterschiedliche Werte für die Vergleichsverbindung A erhalten wurden.

Beispiel A

Kultur: Sommergerste, Sorte "Aramir"
Nachauflauf-Blattbehandlung, Konzentration: 6 mg Wirkstoff pro Gefäß

Tabelle A

| Nr. der chem. Beispiele | relative Wuchshohen (cm) |
|---|---|
| unbehandelt | 100 |
| A | 91,7 |
| 2 | 72,2 |
| 25 | 76,8 |
| 26 | 66,9 |
| 49 | 71,4 |
| 115 | 66,2 |
| 125 | 57,7 |
| 132 | 50,7 |
| 133 | 75,8 |
| 94 | 72,0 |
| Tosylate | |
| unbehandelt | 100 |
| A | 90,2 |
| 99 | 79,5 |
| 102 | 82,8 |
| 105 | 83,3 |
| 107 | 69,8 |
| 109 | 52,4 |
| 110 | 69,8 |
| 111 | 70,0 |
| 112 | 77,9 |
| 113 | 76,3 |

Beispiel B

Kultur: Sommerweizen, Sorte "Kolibri" Nachauflauf-Blattbehandlung, Konzentration: 6 mg Wirkstoff pro Gefäß

Tabelle B

| Nr. der chem. Beispiele | relative Wuchshöhen (cm) |
|---|---|
| unbehandelt | 100 |
| A | 80,2 |
| 99 | 74,6 |
| 107 | 65,1 |
| 110 | 69,8 |
| 111 | 73,0 |

Beispiel C

Kultur: Sommerraps, Sorte "Petranova"
Nachflauf-Blattbehandlung, Konzentration: 6 mg Wirkstoff pro Gefäß

Tabelle C

| Nr. der chem. Beispiele | relative Wuchshöhen (cm) |
|---|---|
| unbehandelt | 100 |
| A | 91,7 |
| 1 | 83,2 |
| 2 | 80,5 |
| 26 | 68,5 |
| 44 | 84,3 |
| 45 | 86,2 |
| 71 | 83,2 |
| 95 | 70,7 |
| 105 | 78,8 |
| 113 | 76,8 |
| Tosylate | |
| unbehandelt | 100 |
| A | 91,6 |
| 95 | 80,3 |
| 102 | 82,4 |
| 107 | 80,3 |
| 109 | 80,7 |
| 110 | 76,2 |
| 111 | 78,2 |
| 112 | 78,2 |
| 113 | 78,2 |

Beispiel D

Kultur: Sommerblumen, Sorte "Sorex"
Nachauflauf-Blattbehandlung, Konzentration: 6 mg Wirkstoff pro Gefäß

Tabelle D

| Nr. der chem. Beispiele | relative Wuchshöhen (cm) |
|---|---|
| unbehandelt | 100 |
| A | 90,6 |
| 1 | 73,2 |
| 19 | 77,5 |
| 25 | 71,6 |
| 26 | 60,4 |
| 44 | 80,0 |
| 45 | 80,0 |
| 77 | 87,4 |
| 95 | 72,7 |
| 112 | 62,0 |
| 121 | 83,5 |
| 94 | 76,2 |
| Tosylate | |
| unbehandelt | 100 |
| A | 80,8 |
| 99 | 69,9 |

Beispiel E

Kultur: Reis, Sorte "Bahia"
Nachauflauf-Blattbehandlung

Tabelle E

| Nr. der chem. Beispiele | Konzentration mg pro Gefäß | relative Wuchshöhen (cm) |
|---|---|---|
| unbehandelt | - | 100 |
| A | 1,5 | 97,5 |
| | 6 | 97,5 |
| 2 | 1,5 | 87,5 |
| | 6 | 75,0 |
| 26 | 1,5 | 95,8 |
| | 6 | 72,8 |
| 49 | 1,5 | 82,5 |
| | 6 | 70,0 |
| Tosylate | | |
| unbehandelt | - | 100 |
| A | 1,5 | 96,9 |
| | 6 | 96,9 |
| 109 | 1,5 | 87,0 |
| | 6 | 57,1 |

Beispiel F

Reiskeimlingstest

In weiteren Untersuchungen wurde junge Reiskeimlinge (Sorte Girona) in einer Nährlösung kultiviert, die die jeweiligen Wirkstoffe in unterschiedlichen Konzentrationen enthielt. Nach sechs Tagen Kultur bei 25°C im Dauerlicht wurde die Wirkstoffkonzentration bestimmt, die das Längenwachstum der zweiten Blattscheide um 50 % vermindert (= $KI_{50}$).

(Details bei W. Rademacher und J. Jung, Berichte aus dem Fachgebiet Herbologie, Heft 24, Seiten 127 bis 134, Universität Hohenheim, 1983).

Die Ergebnisse sind der nachfolgenden Tabelle zu entnehmen.

Tabelle F

| Nr. der chem. Beispiele | $KI_{50}$ (molar) |
|---|---|
| A | $1,5 \times 10^{-2}$ |
| 115 | $6,2 \times 10^{-5}$ |
| 125 | $5,9 \times 10^{-4}$ |
| 132 | $4,4 \times 10^{-5}$ |
| 133 | $5,1 \times 10^{-5}$ |

## Ansprüche

1. Triazolverbindungen der Formeln Ia und Ib

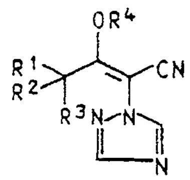

Ia

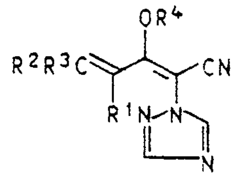

Ib

in denen

$R^1$ Wasserstoff, Cyclopropyl, unsubstituiertes $C_1$-$C_4$-Alkyl oder $C_2$-$C_4$-Alkinyl oder durch Methyl, Ethyl, Ethenyl, Ethinyl, Hydroxyl, Fluor, Chlor, Brom oder $C_1$-$C_4$-Alkoxy substituiertes $C_1$-$C_4$-Alkyl oder $C_2$-$C_4$-Alkinyl bedeutet oder auch für eine $C_1$-$C_4$-Alkoxy-, $C_2$-$C_4$-Alkenyloxy- und $C_2$-$C_4$-Alkinyloxygruppe steht

$R^2$, $R^3$ unabhängig voneinander Wasserstoff, lineares oder verzweigtes $C_1$-$C_3$-Alkyl bedeuten, wobei die Reste $R^2$ und $R^3$ auch zusammen einen gegebenenfalls Brückenglieder aufweisenden carbocyclischen Ring mit bis zu sechs Gliedern in jedem Ringsystem bilden können und mit der Maßgabe, daß in der Formel Ia mindestens zwei der Reste $R^1$-$R^3$ von Wasserstoff verschieden sind und

$R^4$ Wasserstoff, einen Acylrest CO-$R^5$ oder einen Sulfonylrest $SO_2$-$R^5$, wobei $R^5$ jeweils für lineares oder verzweigtes $C_1$-$C_8$ Alkyl oder gegebenenfalls substituiertes Phenyl steht, bedeutet oder ein Metallkation oder Ammonium darstellt.

2. Triazolverbindungen der Formel Ia

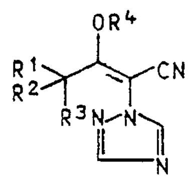

Ia

in der

$R^1$ und $R^2$ für Ethyl,

$R^3$ für Wasserstoff oder Methyl stehen und

$R^4$ die in Anspruch 1 genannte Bedeutung hat.

3. Verfahren zur Herstellung von Azolen der Formel I gemäß Anspruch 1, dadurch gekennzeichnet, daß man ein entsprechendes Säurehalogenid $R^1R^2R^3CCOX$ (X = Halogen) in Anwesenheit einer geeigneten Base oder eines Säurefängers und gegebenenfalls in Gegenwart eines Verdünnungsmittels mit Triazolylacetonitril umsetzt und das Umsetzungsprodukt gegebenenfalls acyliert bzw. in ein Salz überführt oder mit einem Sulfonsäurehalogenid oder -anhydrid zur Reaktion bringt.

4. Verfahren zur Regulierung des Pflanzenwachstums, dadurch gekennzeichnet, daß man den Boden, die Pflanzen oder ihren Samen mit einer Triazolverbindung der Formeln Ia oder Ib, in denen $R^1$-$R^5$ die oben genannte Bedeutung besitzen, behandelt.

5. Mittel zur Regulierung des Pflanzenwachstums, enthaltend eine Triazolverbindung der Formeln Ia oder Ib nach Anspruch 1.

6. Mittel zur Regulierung des Pflanzenwachstums, enthaltend eine Triazolverbindung der Formeln Ia oder Ib nach Anspruch 1 und inerte Zusatzstoffe.

7. Verfahren zur Regulierung des Pflanzenwachstums, dadurch gekennzeichnet, daß man mindestens eine Triazolverbindung der Formel Ia oder Ib nach Anspruch 1 bzw. ein Mittel nach Anspruch 5 oder 6 auf Pflanzen oder deren Lebensraum einwirken läßt.

Europäisches Patentamt

# EUROPÄISCHER RECHERCHENBERICHT

Nummer der Anmeldung

EP 88 10 7592

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 102 163 (CHEVRON RESEARCH CO.) * Seite 1, Zeile 1 - Seite 3, Zeile 5; Beispiel3; Tabelle 1, Verbindungen 11,12,15-17 * | 1,4-7 | C 07 D 249/08<br>A 01 N 43/653 |
| A | EP-A-0 164 088 (BASF AG) * Ansprüche 1,6-9; Seite 11, Verbindungen 50-67 * | 1,4-7 | |
| A | EP-A-0 167 028 (BAYER AG) *,Seite 3, Formel III *& DE - A - 34 23101 (Kat. D) | 1 | |

**RECHERCHIERTE SACHGEBIETE (Int. Cl.4)**

C 07 D 249/00
A 01 N 43/00
C 07 D 521/00

Der vorliegende Recherchenbericht wurde für alle Patentansprüche erstellt

| Recherchenort | Abschlußdatum der Recherche | Prüfer |
|---|---|---|
| BERLIN | 09-08-1988 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)